# EUROPÄISCHE PATENTSCHRIFT

(11) **EP 1 504 734 B1**
(45) Veröffentlichungstag und Bekanntmachung des Hinweises auf die Patenterteilung: **09.11.2005**
(21) Anmeldenummer: 03018049.1
(22) Anmeldetag: 07.08.2003
(51) Int. Cl.: A61F 2/46

(54) **Einsetzinstrument für ein Paar von Schlittenprothesen**
Insertion instrument for sliding prostheses
Instrument d'insertion pour prothèses à glissement

(43) Veröffentlichungstag der Anmeldung: 09.02.2005
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll

(56) Entgegenhaltungen:
- EP-A- 1 099 430
- EP-A- 1 321 116

## Beschreibung

Bekanntlich enthält das Kniegelenk zwei tibio-femorale Gelenkflächenpaare, nämlich ein mediales und ein laterales Gelenkflächenpaar, die jeweils von einem femoralen Kondylus und einer damit zusammenwirkenden, schalenförmigen tibialen Gelenkfläche gebildet sind. Es ist bekannt (EP-A 1099430), die Gelenkflächen der femoralen Kondylen je einzeln durch eine Schlittenprothese zu ersetzen und dafür ein Einsetzinstrument zu verwenden, das ihre genaue gegenseitige Ausrichtung gewährleistet. Die Prothesen werden an dem Instrument mittels Klemmen gehalten, die über eine lösbare Kupplung an einer Tragplatte des Instruments befestigt sind. Sobald bei der Operation die Prothesen den gewünschten Sitz erreicht haben, löst man die Kupplung und zieht das Instrument mit der Tragplatte von den Klemmen ab, die an den Prothesen verbleiben, bis der zu ihrer Befestigung verwendete Zement erhärtet ist. Die lösbare Kupplung besteht aus einem Zapfen an jeder Klemme und zwei Aufnahmeöffnungen in der Tragplatte, in die diese Zapfen genau passend eingesteckt werden können. Sie werden darin mittels Schrauben gesichert, die während der Operation zugänglich bleiben, so daß sie gelöst werden können, wenn das Instrument von den Klemmen abgenommen werden soll. Um Prothesen mit unterschiedlichem, vorbestimmtem Abstand einsetzen zu können, sind unterschiedliche Tragplatten vorgesehen, in denen die Aufnahmeöffnungen für die Klemmenzapfen unterschiedlichen, vorbestimmten Abstand voneinander aufweisen. Dies hat den Nachteil, daß eine Vielzahl verschiedener Instrumente mit unterschiedlichen Tragplatten vorrätig gehalten werden muß. Außerdem möchte der Operateur mitunter den Abstand der Prothesen noch während der Operation an dem dafür verwendeten Einsetzinstrument ändern können. Es wurde deshalb ein weiteres bekanntes Instrument geschaffen (EP-A 1321116), bei welchem für die an den Klemmen vorgesehenen Zapfen in der Tragplatte eine Führung vorgesehen ist, in welcher die Zapfen und damit die Klemmen in LM-Richtung (LM = lateral-medial) mittels einer Gewindespindel verschoben werden können. Dadurch wurde zwar das Ziel der interoperativen Verstellbarkeit des Abstands gelöst; man mußte aber den Nachteil in Kauf nehmen, daß die Klemmen durch die Gewindespindel bleibend an der Tragplatte verankert sind und daß das Instrument nur dadurch von den implantierten Prothesen abgenommen werden kann, daß die Klemmen selbst geöffnet werden, was nicht ganz einfach ist.

Der Erfindung liegt daher die Aufgabe zugrunde, ein Einsetzinstrument für Schlittenprothesen der oben zuerst erläuterten Art zu schaffen, das eine unterschiedliche Abstandseinstellung der Klemmen und deren intraoperative Trennung vom Instrument erlaubt.

Die erfindungsgemäße Lösung liegt in den Merkmalen des Anspruchs 1. Dabei wird vorausgesetzt, daß die Kupplung zwischen den Klemmen und der Tragplatte zumindest an einer der beiden Klemmen in LM-Richtung lageindifferent ist, so daß die betreffende Klemme in unterschiedlichem Abstand von der anderen Klemme eingestellt werden kann. Es ist eine Fixiereinrichtung zum Fixieren der Klemme in einer gewählten Position und zum Blockieren der Kupplung vorgesehen.

Die in LM-Richtung lageindifferente Kupplung kann - ähnlich den bekannten Lösungen - aus einem Zapfen an der Klemme und einem den Zapfen aufnehmenden Langloch in der Tragplatte bestehen. Die Klemme ist an der Tragplatte so geführt, daß ihr lediglich eine seitliche Einstellbewegung (in LM-Richtung) erlaubt ist.

Zweckmäßigerweise sind beide Klemmen je über eine in LM-Richtung lageindifferente Kupplung mit der Tragplatte verbunden und in der gewählten Einstellung fixierbar. Dadurch soll erreicht werden, daß die beiden Klemmen jeweils symmetrisch zur Instrumentenachse angeordnet werden können.

Zum Fixieren der Klemmen in der gewählten Stellung ist nach der Erfindung ein Fixierjoch vorgesehen, dessen Enden quer zur Einstellrichtung und zur Löserichtung der Kupplungen auf je ein mit den Klemmen verbundenes Kupplungsteil drücken, nämlich zweckmäßigerweise auf den Kupplungszapfen. Mit der Tragplatte ist das Fixierjoch mittig durch ein Preßmittel, beispielsweise eine Schraube, verbunden, die intraoperativ betätigbar ist. Dadurch ist es intraoperativ möglich, die Kupplung zu lösen und ggf. nach Änderung des Klemmenabstands wieder zu schließen. Es ist nicht erforderlich, daß das Fixierjoch genau lotrecht zu den Einstell- und Löserichtungen auf den festzuhaltenden Kupplungsteil wirkt. Jedoch ist dies im allgemeinen vorteilhaft.

Das Fixierjoch ist zweckmäßigerweise in LM-Richtung ebenso lageindifferent wie die Kupplung, um die Klemmen in jeder beliebigen Abstandseinstellung festhalten zu können. Wenn man nur bestimmte Abstandseinstellungen zulassen will, beispielsweise solche, die mit bestimmten tibialen Prothesenteilen harmonieren (siehe beispielsweise EP-A 1099430), kann man das Fixierjoch auch so ausbilden, daß es nur in den gewünschten Abstandseinstellungen auf die Kupplungsteile der Klemmenzapfen einwirken kann. Zum Beispiel kann das Joch mit Vorsprüngen oder Einschnitten versehen sein, die zu Ausnehmungen oder Vorsprüngen der Zapfen passen. Es können auch mehrere austauschbare Fixierjoche vorgesehen sein, von denen jedes nur bei einem vorbestimmten Klemmenabstand mit den zugehörigen Klemmenzapfen zusammenwirken kann und dadurch als Abstandslehre wirkt. Schließlich besteht auch die Möglichkeit, zusätzlich zu dem in LM-Richtung lageindifferenten Fixierjoch eine Abstandslehre vorzusehen. Die Abstandslehre ist einerseits zur Fixierung an der Tragplatte in vorbestimmter Stellung ausgebildet. Andererseits weist sie Ausnehmungen oder Vorsprünge auf, die mit den Klemmenzapfen oder Teilen derselben lagebestimmend zusammenwirken.

Die Erfindung wird im folgenden näher unter Bezugnahme auf die Zeichnung erläutert, die ein vorteilhaftes Ausführungsbeispiel veranschaulicht. Es zeigen:
- Fig. 1: eine Gesamtansicht des Instruments,
- Fig. 2: wesentliche Teile des Instrumentenkopfs in teilweise auseinandergezogenem Zustand,
- Fig. 3: das Fixierjoch und
- Fig. 4: einen Teilschnitt durch das Instrument.

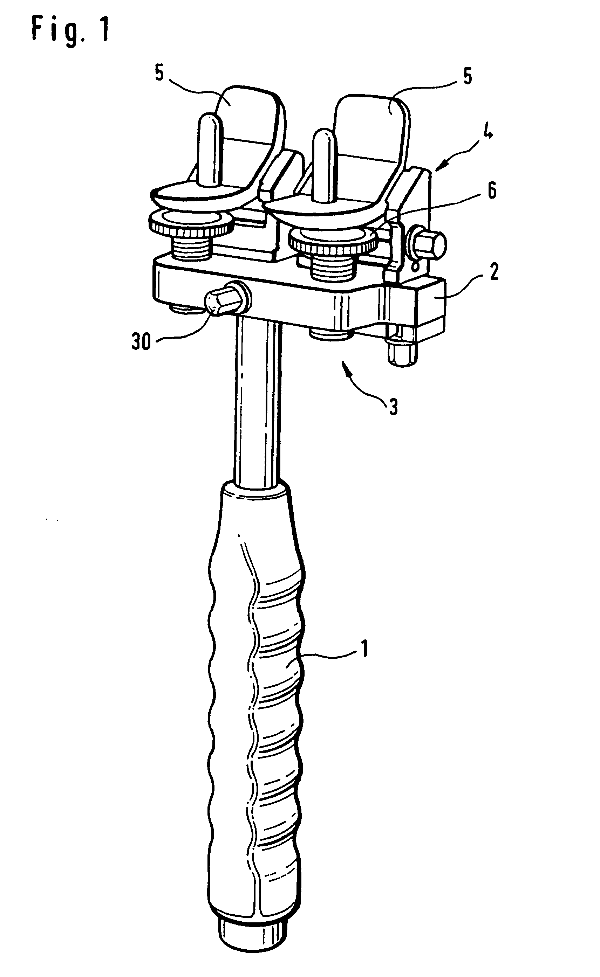

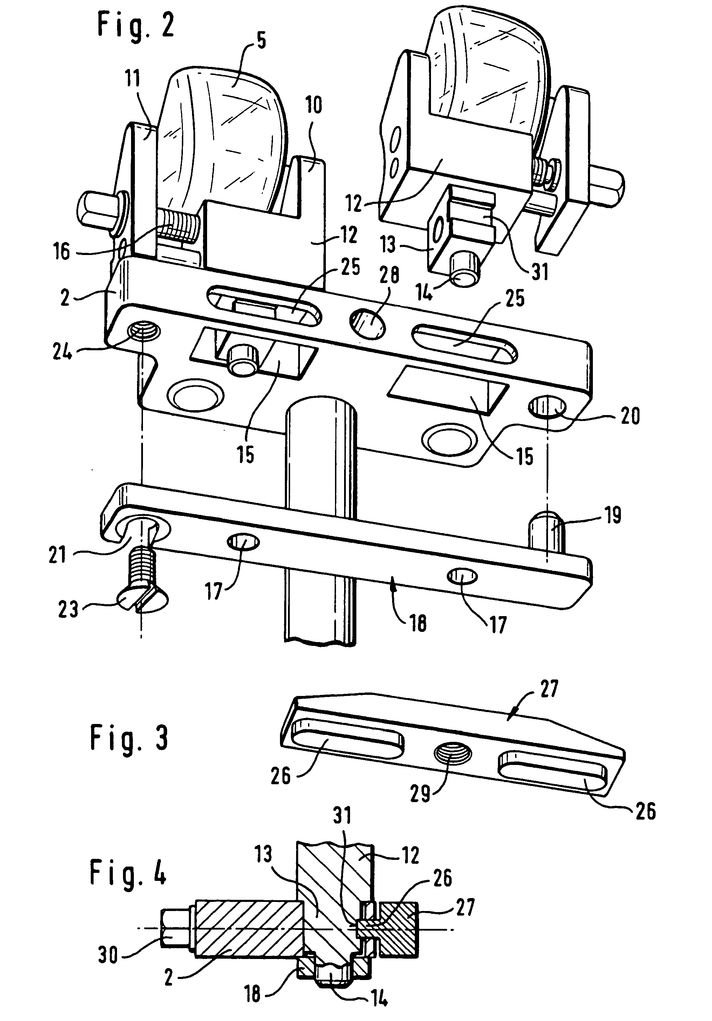

In Fig. 1 sieht man die Schlittenprothesen 5 schräg von dorsal-medial und in Fig. 2 schräg von ventral. Dieselben Richtungsbezeichnungen werden im folgenden auch auf das Instrument angewendet.

An einem Stiel mit Griff 1 ist die Tragplatte 2 des Instrumentenkopfs 3 befestigt. Die Tragplatte 2 trägt zwei Klemmen 4 für zwei Schlittenprothesen 5 sowie zwei Gleitflächenstützen 6.

Die Gleitflächenstützen 6 bestehen aus einem in der Tragplatte 2 gehaltenen Gewindeschaft und einem Kopf mit einem Schlagpolster aus steifelastischem Kunststoff. Durch Verdrehen lassen sie sich so einstellen, daß ihre Oberseite stützend an der Gleitfläche des in der zugehörigen Klemme 4 befindlichen Prothesenteils anliegt.

Jede Klemme 4 umfaßt eine erste Klemmbacke 10 und eine zweite Klemmbacke 11. Der Fuß 12 der ersten Klemmbacke 10 sitzt vollflächig auf der Oberseite der Tragplatte 2 auf und wird von ihr in später zu erläuternder Weise gehalten. Die zweite Klemmbacke 11 ist vom Fuß 12 der ersten Klemmbacke 10 parallel verschiebbar gehalten. Der Abstand der Klemmbacken 10, 11 voneinander ist mittels einer Gewindespindel 16 einstellbar.

Beide Klemmbacken 10, 11 tragen Vorsprünge, die mit entsprechenden Ausnehmungen der Prothesen 5 zusammenwirken, wenn diese in die Klemmen 4 eingesetzt sind. Dadurch werden die Prothesen gegenüber dem Instrument eindeutig positioniert. Einzelheiten ihrer Konstruktion und Wirkungsweise lassen sich der EP-A-1099430 entnehmen.

Auf der Unterseite jedes Klemmenfußes 12 springt ein Zapfen 13 mit rechteckigem Profil vor, der an seiner Unterseite einen kleinen zylindrischen Ansatz 14 trägt. Die Tragplatte 2 enthält zwei Langlöcher 15, in die je ein rechteckiger Zapfen 13 genau hineinpaßt. Die Langlöcher 15 und die Zapfen 13 bilden zusammenwirkende Führungen in LM-Richtung des Instruments. Jede Klemme 4 kann daher unterschiedliche Positionen längs ihrer in LM-Richtung verlaufenden Verschiebelinie auf der Tragplatte 2 einnehmen. Die Rechteckzapfen 13 und die Langlöcher 15 bilden die oben erwähnte lösbare Kupplung zwischen den Klemmen 4 und der Tragplatte 2.

Der im Profil rechteckige Zapfen 13 ist im montierten Zustand gänzlich von dem zugehörigen Langloch 15 aufgenommen. Lediglich der kleine zylindrische Ansatz 14 ragt auf der Unterseite aus der Tragplatte 2 hervor. Er wirkt zusammen mit je einer von zwei Bohrungen 17 in einer Abstandslehre 18, die auf der Unterseite der Tragplatte 2 befestigbar ist. Am einen Ende weist sie einen Zapfen 19 auf, der in eine passende Bohrung 20 der Tragplatte einsetzbar ist, und am anderen Ende eine seitlich offene Bohrung 21, die mit einer Schraube 23 in einer Gewindebohrung 24 der Tragplatte zusammenwirkt. Es steht eine Mehrzahl von Abstandslehren 18 zur Verfügung deren symmetrisch angeordnete Bohrungen 17 unterschiedlichen Abstand voneinander haben.

Auf der dorsalen Seite der Tragplatte 2 ist diese im Bereich der Langlöcher 15 mit langgestreckten Ausschnitten 25 versehen. Diesen Ausschnitten 25 entsprechen Vorsprünge 26 an einem Fixierjoch 27, das an der dorsalen Seite der Tragplatte 2 mit in die Ausschnitte 25 hineinragenden Vorsprünge 26 angebracht wird. Es wird daran mittels einer die Bohrung 28 der Tragplatte durchdringenden und in die mittig angeordnete Gewindebohrung 29 des Fixierjochs 27 fassende Gewindeschraube 30 gehalten, deren Kopf auf der ventralen Seite der Tragplatte 2 zugänglich ist. Wenn das Fixierjoch 27 mittels der Schraube 30 gegen die Rückseite der Tragplatte gezogen wird, dringen seine Vorsprünge 26 durch die Ausschnitte 25 in die Langlöcher 15 ein (siehe Fig. 4) und drücken auf die dort befindlichen Rechteckzapfen 13 der Klemmen 4, wodurch diese in ihrer jeweiligen Stellung fixiert werden. Die Rechteckzapfen 13 sind an der dorsalen Seite mit einer quer verlaufenden, flachen Nut 31 versehen, in die die Stirn der Vorsprünge 26 des Fixierjochs 27 eingreift.

Das Instrument wird in folgender Weise benutzt. Gemäß dem gewünschten Prothesenabstand wird eine Abstandslehre 18 ausgewählt und mit der Unterseite der Tragplatte 2 verbunden. Das Fixierjoch 27 wird mittels der Schraube 30 an der Rückseite der Tragplatte 2 lose befestigt, so daß seine Vorsprünge 26 in die Ausschnitte 25, aber noch nicht in den Bereich der Langlöcher 15 eindringen. Sodann werden die Klemmen 4 eingesetzt, so daß ihre Rechteckzapfen 13 in den Langlöchern 15 und ihre kleinen zylindrischen Ansätze 14 in den Bohrungen 17 der Abstandslehre 18 liegen. Danach zieht man das Fixierjoch 27 mit der Schraube 30 fest. Die Klemmen sind nun auf der Oberseite der Tragplatte fest und im gewünschten Abstand gehalten. Danach werden die Prothesen 5 in die Klemmen 4 eingesetzt. Gewünschtenfalls kann dies auch schon geschehen, bevor die Klemmen 4 mit der Tragplatte 2 verbunden werden.

Will man den Abstand der Prothesen ändern, so nimmt man die Abstandslehre 18 ab, lockert vorsichtig die Schrauben 30, so daß die Klemmen 4 in LM-Richtung an der Tragplatte 2 verschiebbar werden. Wenn die Vorsprünge 26 noch in die Nut 31 eingreifen, sind sie gegen Herausfallen gesichert. Es kann nun eine andere Abstandslehre 18 mit der Tragplatte verbunden werden, wobei gleichzeitig die Klemmen 4 so verschoben werden, daß ihre zylindrischen Ansätze 14 in die neu positionierten Löcher 17 der Abstandslehre 18 eingreifen. Danach wird das Fixierjoch 27 wieder angezogen.

Nachdem die Prothesen mittels des Instruments am Patienten die gewünschte Stellung erhalten haben, wird die Kupplung des Instruments zwischen den Klemmen 4 und der Tragplatte 2 gelöst. Zu diesem Zweck wird die Schraube 30 so weit gelöst, daß das Instrument mit der Tragplatte 2 von den Klemmen in kaudaler Richtung abgezogen werden kann. Dies kann geschehen, bevor ggf. verwendeter Zement erhärtet ist.

## Patentansprüche

1. Einsetzinstrument für ein Paar von Schlittenprothesen (5), das an einer Tragplatte (2) zwei mit vorbestimmtem Abstand voneinander angeordnete Klemmen (4) für die Schlittenprothese (5) trägt, die über eine lösbare Kupplung (13, 15) davon abnehmbar sind, wobei zum Einstellen unterschiedlicher Klemmenabstände die Kupplung (13, 15) zumindest an einer der beiden Klemmen (4) in LM-Richtung lageindifferent ist, **dadurch gekennzeichnet, daß** eine Fixiereinrichtung (27) zum Fixieren der Klemme (4) in einer gewählten Position und zum Blockieren der Kupplung vorgesehen ist.

2. Instrument nach Anspruch 1, **dadurch gekennzeichnet, daß** die in LM-Richtung lageindifferente Kupplung aus einem Zapfen (13) an der Klemmen (4) und einem den Zapfen (13) aufnehmenden Langloch (15) in der Tragplatte besteht.

3. Instrument nach Anspruch 1 oder 2, **dadurch gekennzeichnet, daß** beide Klemmen (4) über je eine in LM-Richtung lageindifferente Kupplung (13, 15) mit der Tragplatte (2) verbunden sind.

4. Instrument nach Anspruch 3, **dadurch gekennzeichnet, daß** die Fixiereinrichtung von einem Fixierjoch (27) gebildet ist, dessen Enden quer zu der Einstellrichtung und der Löserichtung der Kupplungen (13, 15) auf je ein mit den Klemmen (4) verbundenes Kupplungsteil (13) drücken und das mittig durch ein vorzugsweise intraoperativ betätigbares Preßmittel (30) mit der Tragplatte (2) verbunden ist.

5. Instrument nach Anspruch 4, **dadurch gekennzeichnet, daß** das Fixierjoch (27) in LM-Richtung ebenso lageindifferent ist wie die Kupplung.

6. Instrument nach Anspruch 5, **dadurch gekennzeichnet, daß** eine zusätzliche, wechselbare Abstandslehre (18) vorgesehen ist.

7. Instrument nach Anspruch 6, **dadurch gekennzeichnet, daß** die Abstandslehre (18) mit der Halteplatte (2) verbindbar ist.

8. Instrument nach Anspruch 4, **dadurch gekennzeichnet, daß** das Fixierjoch (27) eine Abstandslehre bildet.

## Claims

1. Insertion instrument for a pair of sliding prostheses (5), said instrument having, on a support plate (2), two clamps (4) which are arranged at a predetermined spacing from one another and receive the sliding prostheses (5), which can be removed from them via a releasable coupling (13, 15), wherein, in order to set different clamp spacings, the coupling (13, 15) is provided in any position in the LM direction, at least on one of the two clamps (4), **characterized in that** a fixing means (27) is provided for fixing the clamp (4) in a chosen position and for blocking the coupling.

2. Instrument according to Claim 1, **characterized in that** the coupling provided in any position in the LM direction consists of a plug (13) on the clamps (4), and of an elongate hole (15) arranged in the support plate and receiving the plug (13).

3. Instrument according to Claim 1 or 2, **characterized in that** both clamps (4) are connected to the support plate (2) via in each case a coupling (13, 15) provided in any position in the LM direction.

4. Instrument according to Claim 3, **characterized in that** the fixing means is formed by a fixing yoke (27) whose ends, transverse to the adjustment direction and release direction of the couplings (13, 15), press on a coupling part (13) connected to the clamps (4), and which is connected centrally to the support plate (2) by a pressing means (30) which can preferably be actuated during the operation.

5. Instrument according to Claim 4, **characterized in that** the fixing yoke (27) is, like the coupling, provided in any position in the LM direction.

6. Instrument according to Claim 5, **characterized in that** an additional, exchangeable spacing gauge (18) is provided.

7. Instrument according to Claim 6, **characterized in that** the spacing gauge (18) can be connected to the holding plate (2).

8. Instrument according to Claim 4, **characterized in that** the fixing yoke (27) forms a spacing gauge.

## Revendications

1. Instrument d'insertion pour une paire de prothèses à glissement (5), qui porte sur une plaque de support (2) deux pinces (4), disposées à distance déterminée l'une de l'autre, pour la prothèse à glissement (5), lesquelles pinces peuvent être retirées de celle-ci par un accouplement (13, 15) séparable, l'accouplement (13, 15) étant indifférent à la position dans la direction LM, au moins sur l'une des deux pinces (4), pour le réglage d'écartements différents des pinces, **caractérisé en ce qu'**il est prévu un dispositif de fixation (27) pour fixer la pince (4) dans une position sélectionnée,et pour bloquer l'accouplement.

2. Instrument selon la revendication 1, **caractérisé en ce que** l'accouplement indifférent à la position dans la direction LM est constitué d'un axe (13) sur les pinces (4) et d'un trou oblong (15) recevant l'axe (13), dans la plaque de support.

3. Instrument selon la revendication 1 ou 2, **caractérisé en ce que** les deux pinces (4) sont reliées à la plaque de support (2) chacune par un accouplement (13, 15) indifférent à la position dans la direction LM.

4. Instrument selon la revendication 3, **caractérisé en ce que** le dispositif de fixation est formé par une traverse de fixation (27) dont les extrémités pressent chacune, transversalement à la direction de réglage et à la direction de déverrouillage des accouplements (13, 15), sur un élément d'accouplement (13) relié aux pinces (4), ainsi qu'au centre, à la plaque de support (2), par un moyen de pression (30) pouvant être actionné de préférence pendant l'opération.

5. Instrument selon la revendication 4, **caractérisé en ce que** la traverse de fixation (27) est également indifférente à la position dans la direction LM, comme l'accouplement.

6. Instrument selon la revendication 5, **caractérisé en ce qu'**il est prévu une jauge d'écartement (18) échangeable supplémentaire.

7. Instrument selon la revendication 6, **caractérisé en ce que** la jauge d'écartement (18) peut être reliée à la plaque de support (2).

8. Instrument selon la revendication 4, **caractérisé en ce que** la traverse de fixation (27) forme une jauge d'écartement.
